# EUROPEAN PATENT APPLICATION

(11) **EP 2 499 977 A2**
(43) Date of publication of application: **19.09.2012**
(21) Application number: 10830210.0
(22) Date of filing: 15.11.2010
(51) Int. Cl.: A61B 17/04, A61B 19/00

(54) **SURGICAL DEVICE AND MEDICAL NEEDLE MODULE HAVING INDICATION FUNCTION**

(30) Priority: 13.11.2009 KR 20090109891
(71) Applicant: Rimscience Co., Ltd., Seoul 156-881 (KR)
(72) Inventor: YOON, Sang Jin, Seoul 137-925 (KR)
(74) Representative: Zardi, Marco
(86) International application number: PCT/KR2010/008033
(87) International publication number: WO 2011/059278

(57) **Abstract**

The present invention relates to a surgical device and a medical needle module having an indication function. According to one aspect of the present invention, the medical needle module comprises a medical needle, a light channel unit, and a light radiation unit, wherein the light channel unit provides a light channel for allowing light from the light radiation unit to indicate a position in which the medical needle is to be used.

## Description

### TECHNICAL FIELD

The present invention relates to a surgical device and a medical needle module having an indication function. More particularly, the present invention relates to a technique for enabling a medical needle or a surgical device used by a practitioner such as doctor or the like to perform an indication function during a medical practice.

### BACKGROUND ART

A medical needle is used for various purposes. That is, medical needles range from a surgical needle used together with a suture in order to suture tissues such as serosae, muscles, fasciae, skin, blood vessels or the like of a human body to a needle used for various medical practices, various medical assistant practices, blood-gathering and the like by pressing, pricking, or penetrating a part of a patient's or an animal's body.

### DISCLOSURE

### TECHNICAL PROBLEM

Conventionally, in using a medical needle, especially in using the medical needle in the body, it is a fact that the position of a body part with which the medical needle is to be brought into contact is estimated and adjusted, or the part in which the medical needle has been used is examined again, totally depending on the practitioner's eye measurement. Thus, when the practitioner lacks experience or makes a mistake, a medical needle may be erroneously used, causing problems such that the medical practice is prolonged in a simple case and an emergency occurs in a serious case.

Therefore, in order to address the above problems, the inventor of the present application has devised a medical needle module and a surgical device having an indication function, and thus proposes them through the present disclosure.

### TECHNICAL SOLUTION

It is, therefore, an object of the present invention to solve all the aforementioned problems of the prior art.

It is another object of the present invention to provide a novel medical needle module capable of allowing a medical needle to be precisely used.

It is still another object of the present invention to provide a novel surgical device capable of allowing a medical needle to be precisely used.

It is still another object of the present invention to allow a required indication to be carried out on a body part during a surgical operation.

### ADVANTAGEOUS EFFECTS

In accordance with the present invention, a novel medical needle module capable of allowing a medical needle to be precisely used is provided.

In accordance with the present invention, a novel surgical device capable of allowing a medical needle to be precisely used is provided.

In accordance with the present invention, it enables a required indication to be carried out on a body part during a surgical operation.

### DESCRIPTION OF DRAWINGS

The above objects and features of the present invention will become apparent from the following description of the preferred embodiments given in conjunction with the accompanying drawings, in which:
Fig. 1 is a view schematically illustrating a portion of a medical needle module 100 in accordance with an embodiment of the present invention.
Fig. 2 is a view schematically illustrating a portion of a medical needle module 100 in accordance with another embodiment of the present invention.
Fig. 3 is a view schematically illustrating a surgical device (i.e., a device for controlling a surgical needle) configured with a combination of the medical needle module 100 and an indication module 200 in accordance with an embodiment of the present invention.
Fig. 4 is a view schematically illustrating that a surgical device 300 forms an indication pattern through a diffraction unit 350 in accordance with an embodiment of the present invention.

### BEST MODE

Representative configurations for achieving the aforementioned objects of the present invention are presented as follows.

In accordance with one aspect of the present invention, there is provided a medical needle module, comprising: a medical needle; a light channel unit; and a light irradiation unit, wherein the light channel unit provides a light channel for allowing light from the light irradiation unit to indicate a position in which the medical needle is to be used.

In accordance with another aspect of the present invention, there is provided a surgical device, comprising: a medical needle module; and an indication module, wherein the indication module comprises a plurality of indication units, and the medical needle module can move with respect to the indication module.

In accordance with another aspect of the present invention, there is provided a surgical device, comprising: a light irradiation unit; and a diffraction unit, wherein the diffraction unit forms an indication pattern on a body part with light irradiated from the light irradiation unit, and the diffraction unit is detachable.

### MODE FOR INVENTION

In the following detailed description, reference is made to the accompanying drawings that show, by way of illustration, specific embodiments in which the present invention may be practiced. These embodiments are described in sufficient detail to enable those skilled in the art to practice the present invention. It is to be understood that the various embodiments of the present invention, although different, are not necessarily mutually exclusive. For example, a particular feature, structure or characteristic described herein in connection with an embodiment may be implemented within other embodiments without departing from the spirit and scope of the present invention. In addition, it is to be understood that the locations or arrangements of individual elements within each disclosed embodiment may be modified without departing from the spirit and scope of the present invention. The following detailed description is, therefore, not to be taken in a limiting sense, and the scope of the present invention is defined only by the appended claims, appropriately interpreted, along with the full range of equivalents to which the claims are entitled. In the drawings, like numerals refer to the same or similar elements throughout the several views.

Hereinafter, various preferred embodiments of the present invention will be described in detail with reference to the accompanying drawings so that a person skilled in the art can easily practice the present invention.

### First Embodiment

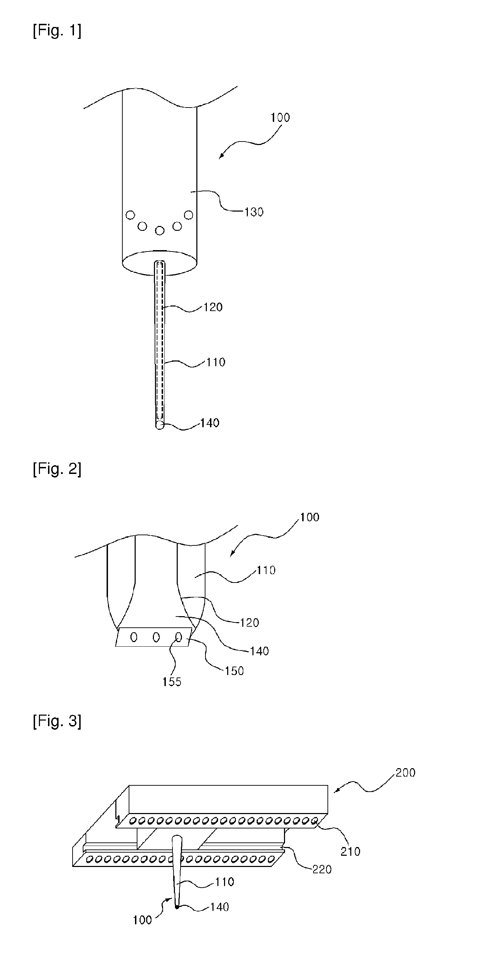

Fig. 1 is a view schematically illustrating a portion of a medical needle module 100 in accordance with an embodiment of the present invention.

As shown in Fig. 1, the medical needle module 100 may include a medical needle 110, a light channel unit 120, and a light irradiation unit 130.

First, the medical needle 110 in accordance with an embodiment of the present invention may have a linear shape as illustrated or may have a slightly curved shape. The medical needle 110 may be made of various materials such as metal, e.g., stainless steel, wood, a synthetic resin, rubber, ceramic, or the like.

The medical needle 110 in accordance with an embodiment of the present invention may include a tip portion and a body part extended from the tip portion and including the light channel unit 120 therein as illustrated. In consideration of certain stiffness that is generally required for the medical needle 110, it is preferred that the medical needle 110 may have a section width of the thickest portion surrounding the light channel unit 120 ranging from about 0.5 mm to 1.5 mm, which may be different depending on the materials. Of course, for good penetration characteristics, it is preferred that the medical needle 110 may be configured to taper toward the tip portion such that a section width of a lower portion is smaller than that of an upper portion.

Next, the light channel unit 120 in accordance with an embodiment of the present invention will be described. The light channel unit 120 may serve to provide a path to the tip portion 140 (i.e., to the interior of the tip portion of the medical needle 110) to light irradiated from the light irradiation unit 130 as illustrated such that the light irradiated from the light irradiation unit 130 is cast upon a body part requiring a light indication in accordance with the present invention. Preferably, the interior of the light channel unit 120 may be coated with a material (e.g., a known light transmissive material or an optical fiber material) advantageous for light reflection in order to cope with such situation that the light irradiated from the light irradiation unit 130 has poor straightness. More preferably, the tip portion 140 of the light channel unit 120 may further include a condensing lens (not shown) to enhance the straightness of light irradiated from the light channel unit 120 to the outside. The condensing lens may be a focusing lens or a collimator.

Finally, the light irradiation unit 130 in accordance with an embodiment of the present invention will be described. The light irradiation unit 130 may perform a function of generating light from electrical energy and irradiating light. Preferably, such light may be a type of light appropriate to be irradiated to the outside through the light channel unit 120 (e.g., laser light) due to its excellent straightness. Thus, the light irradiation unit 130 may include a known laser pointer (not shown).

The laser pointer is a device for irradiating bright light to a certain portion having a small area to indicate or highlight the corresponding portion. The laser pointer is a device capable of emitting visible colored light having high straightness with only a small output power ranging from about 5 mW to 1000 mW. Thus, the laser pointer may be appropriate for a practitioner or the like to properly observe the light having such a small power as not to harm a body part (especially a weak part of a human body). Various known laser pointers may be applied to the present invention without limitation, and most preferably, a low-priced DPSS laser pointer employing an infrared diode laser module (not shown) may be applied to the present invention. In particular, when the color of light from the DPSS laser pointer is green or blue, the practitioner can accurately recognize a light-irradiated portion without being confused with a tissue or blood observed by the practitioner.

### Second Embodiment

Fig. 2 is a view schematically illustrating a portion of a medical needle module 100 in accordance with another embodiment of the present invention.

The configuration of the medical needle module 100 in accordance with the present embodiment is similar to that described in the first embodiment. In this embodiment, however, it should be noted that a diffraction unit 150 is further disposed in the tip portion 140 of the light channel unit 120. As illustrated, the diffraction unit 150 may include two or more diffraction slits 155.

Also, according to the present embodiment, light from the light irradiation unit 130 may be irradiated to the outside through the tip portion 140 after passing through the light channel unit 120. Here, such light may pass through the diffraction slits 155 of the diffraction unit 150 and show a certain indication pattern (a diffraction pattern) on the body part to which the light is irradiated.

Preferably, the diffraction unit 150 may be configured to be detachably attached to the tip portion 140. Thus, the practitioner may see a desired indication pattern by replacing the diffraction unit 150 when he/she wants.

Further, the diffraction unit 150 may have a slit pattern to show a two-dimensional indication pattern, rather than one-dimensional indication pattern. Typically, such a slit pattern may be a pattern having a circumferential shape, a concentric circular shape, or a lattice shape.

In accordance with the present embodiment, when the practitioner uses the medical needle 110 in suturing or the like, he/she may compare and check the interval of the needle stitches already sutured by him/her with the interval of the indication pattern. In other cases, when the practitioner uses the medical needle 110, he/she may accurately measure a distance between a body part indicated by the medical needle 110 and another body part.

### Third Embodiment

Fig. 3 is a view schematically illustrating a surgical device (i.e., a device for controlling a surgical needle) configured with a combination of the medical needle module 100 and an indication module 200 in accordance with an embodiment of the present invention.

As illustrated, the indication module 200 in accordance with an embodiment of the present invention may include a plurality of indication units 210 and a medical needle module guide 220.

First, the plurality of indication units 210 in accordance with an embodiment of the present invention may be preferably embedded in the medical needle module guide 220 at regular intervals as illustrated. Each of the plurality of indication units 210 may include a light irradiation unit (not shown) similar to that described in the first embodiment. Further, preferably, each light irradiation unit may be slightly inclined in a direction indicated by the medical needle 110 in order to irradiate light close to the body part indicated by the medical needle 110.

Meanwhile, the medical needle module guide 220 in accordance with an embodiment of the present invention may have a guide structure for guiding the medical needle module 100. Thus, the medical needle module 100 may be configured to move along the medical needle module guide 220 on the body part in which the medical needle module 100 can be used. For example, the medical needle module 100 may further include a wheel (not shown) or a slider (not shown) for such movement.

Finally, it should be appreciated that, in configuring the surgical device in accordance with the present embodiment, an ordinary needle which does not have such an indication function may also be employed in place of the medical needle module 100.

Meanwhile, the inventor of the present application has invented various novel suturing apparatuses using a surgical needle as one of primary components, and such suturing apparatuses may have been already disclosed or will be disclosed in Korean Patent Application No. 2009-37128, PCT International Application No. PCT/KR2009/003419, PCT International Application No. PCT/KR2010/005851, PCT International Application No. PCT/KR2010/005852, and PCT International Application No. PCT/KR2010/006448 (each of the disclosures of the above applications should be regarded as being incorporated herein by reference in its entirety). The inventor of the present application submits that the foregoing suturing apparatuses can be considered as examples of the apparatuses which use the medical needle module or the surgical device in accordance with the present invention.

### Fourth Embodiment

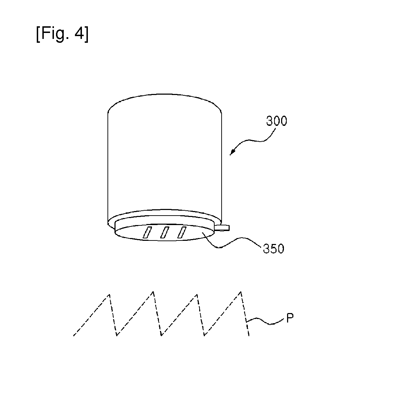

Fig. 4 is a view schematically illustrating that a surgical device 300 forms an indication pattern through a diffraction unit 350 in accordance with an embodiment of the present invention.

As shown in Fig. 4, the surgical device 300 including a light irradiation unit (not shown) may irradiate light such that the light passes through the diffraction unit 350, which is detachably attached to the surgical device 300 as the practitioner requires and may have various patterns of slits, to allow an indication pattern P to appear on a location spaced apart by a certain distance therefrom (preferably, a body part or the like).

The indication pattern P may be variably formed according to the slit patterns of the diffraction unit 350. For example, the indication pattern P may be a pattern P for guiding zigzag suturing as mentioned in the PCT International Application No. PCT/KR2009/003419. Of course, various patterns may be formed to guide suturing other than that mentioned in the PCT International Application No. PCT/KR2009/003419.

### Application of the Invention

In accordance with an application of the present invention, the medical needle module or the surgical device in accordance with the present invention may be employed as a component of a surgical robot system (not shown) increasingly used for various operations. That is, the surgical robot system including the medical needle module or the surgical device in accordance with the present invention may have a reinforced indication function capable of enhancing convenience of practitioners or safety of operations.

While embodiments of the invention has been shown and described, it will be understood by those skilled in the art that various changes and modifications may be made without departing from the spirit and the scope of the invention as defined in the following claims.

## Claims

1. A medical needle module, comprising:
a medical needle;
a light channel unit; and
a light irradiation unit,
wherein the light channel unit provides a light channel for allowing light from the light irradiation unit to indicate a position in which the medical needle is to be used.

2. The medical needle module as claimed in claim 1, wherein the medical needle surrounds the light channel unit.

3. The medical needle module as claimed in claim 1, wherein the interior of the light channel unit is coated with a light transmissive material or an optical fiber material.

4. The medical needle module as claimed in claim 1, wherein the light channel unit includes a condensing lens at a tip portion thereof.

5. The medical needle module as claimed in claim 1, wherein the light channel unit includes a diffraction unit at a tip portion thereof.

6. The medical needle module as claimed in claim 5, wherein the diffraction unit comprises a plurality of diffraction slits.

7. The medical needle module as claimed in claim 1, wherein the light irradiation unit comprises a laser pointer.

8. The medical needle module as claimed in claim 7, wherein the laser pointer irradiates light of green or blue color.

9. A surgical device, comprising:
a medical needle module; and
an indication module,
wherein the indication module comprises a plurality of indication units, and
the medical needle module can move with respect to the indication module.

10. A surgical device, comprising:
a light irradiation unit; and
a diffraction unit,
wherein the diffraction unit forms an indication pattern on a body part with light irradiated from the light irradiation unit, and
the diffraction unit is detachable.
